# EUROPEAN PATENT SPECIFICATION

(11) **EP 2 001 384 B1**
(45) Date of publication and mention of the grant of the patent: **26.08.2009**
(21) Application number: 06745253.2
(22) Date of filing: 31.03.2006
(51) Int. Cl.: A61B 18/14

(54) **DEVICE FOR THE CONTROLLED THERMAL ABLATION OF TUMORS BY MEANS OF HIGH-FREQUENCY ELECTROMAGNETIC ENERGY**
VORRICHTUNG ZUR KONTROLLIERTEN WÄRMEABLATION VON TUMOREN DURCH ELEKTROMAGNETISCHE HOCHFREQUENZ-ENERGIE
DISPOSITIF D'ABLATION THERMIQUE CONTRÔLÉE DE TUMEURS AU MOYEN D'ÉNERGIE ÉLECTROMAGNÉTIQUE HAUTE FRÉQUENCE

(43) Date of publication of application: 17.12.2008
(73) Proprietor: Breval S.R.L., 20145 Milano (MI) (IT)
(72) Inventor: GARBAGNATI, Giberto, I-20129 MILANO MI (IT)
(74) Representative: Concone, Emanuele
(86) International application number: PCT/IT2006/000211
(87) International publication number: WO 2007/113867

(56) References cited:
- EP-A- 1 344 497
- EP-A- 1 634 542
- WO-A-20/06031541
- DE-A1- 2 124 684
- US-A1- 2002 120 260
- US-A1- 2004 133 254
- US-A1- 2004 172 058
- US-A1- 2004 230 316
- US-A1- 2005 096 638
- US-A1- 2005 182 449

## Description

The present invention relates to a device and a method for the treatment of tumors by means of thermal ablation (TA) induced by electromagnetic energy, e. g. in the radiofrequencies (RF) or in the microwaves (MW) range, and particularly to a device and a method for the TA which allows to obtain lesions having a large volume and a predictable and controllable shape.

It is known that the TA procedure induced by electromagnetic energy essentially consist of inserting into a tumoral mass an electrode that, being supplied with electromagnetic energy at a suitable frequency, leads to the generation of heat in the surrounding tumoral tissues, thus causing their coagulative necrosis. The electrode, being generally placed at the end of a needle or a catheter, is percutaneously introduced in the mass of the tumor and it is guided by means of echography or other visualization techniques known in the art. This procedure has proved to be effective for the ablation of tumors of the liver and it has recently been suggested for the ablation of tumors of lung, kidney and other parenchymal organs.

One of the major problems of this kind of procedure consists in the difficulty of destroying tumoral masses having a diameter that is larger than 3 cm. The main reason is that the energy delivered through the electrode inserted in the tumoral mass can not be indefinitely increased. In fact, if on one hand the delivery of high power allows to increase the size of the thermal lesion, on the other hand it causes a rapid dehydration of the tissues closest to the electrode with the consequent impossibility of delivering further energy to the surrounding tissues.

Another problem of the known art is that controlling the shape of the generated thermal lesion is not possible, resulting in the risk of generating thermal lesions poorly corresponding to the shape of the tumor.

Devices and methods for increasing the volume of the thermal lesion in the tumoral mass are already known, consisting of infusing a conductive liquid therein, which transmits energy all around due to its electric conductivity. For instance, patent US 6.911.019 discloses a catheter provided with an helicoidal needle that is inserted in the tumoral mass in order to create an helicoidal cavity being infused with a conductive liquid. The object is to create a channel with a prescribed shape in order to control the size of the thermal lesion. However, this method has the drawback of generating a thermal lesion having an irregular shape and a volume being difficult to predict due to the uncontrollable distribution of the conductive liquid into the tissues.

In patent application US 20040006336 a device is disclosed showing a hollow electrode that allows to improve the infusion of the conductive liquid into the tissue. Also this device exhibits the drawback of not allowing the control of the distribution of the conductive liquid into the tissues, that is the size of the zone being subject to the TA.

The same drawback is also present in the device disclosed in US 2005/096638 relating to a thermal ablation device for the treatment of obesity. In one embodiment, the device comprises an ablation probe including an electrode connected to a hugh frequency electromagnetic energy generator and an expandable membrane suitable for being expanded by a fluid injected therein. The membrane is made of a semipermeable material in order to allow the fluid to leak at a relatively low flow rate thus contacting the tissues surrounding the ablation probe. The fluid is electrically conductive and serves to increase the surface area of the electrode and to evenly distribute heat generated by the electrode across the target tissues.

Object of the present invention is thus to provide a device and a method for the TA being free from the above-mentioned drawbacks, being suitable for increasing the volume of the thermal lesion to the utmost and being suitable for giving it a shape that is as round as possible. Such an object is achieved with the device for TA according to the present invention, whose characteristics are specified in claim 1. Further characteristics of such a device are specified in the dependent claims. In the subsequent claims the characteristics of the method for TA according to the present invention are specified.

Thanks to the use of a substance being electrically conductive, keeping the tissue hydrated around the active part of the electrode and the impedance of the system constantly low, combined with the use of an expandable membrane, locally pressing the tissues to be treated, it is possible to transfer an adequate power level to the tumoral tissue wherein the electrode is inserted, for a much longer time without being limited by the dehydration of the tissues surrounding the electrode.

One advantage of the device and the method for the TA according to the present invention, is that the shape and the volume of the generated thermal lesions are regular and predictable in an extremely precise way. In fact the above-mentioned substance is injected inside a semi-permeable and expandable membrane and closely contacts the tissues surrounding the device while remaining enclosed in the known volume of the membrane.

Another advantage provided by the device and the method for the TA according to the present invention is that the extraction of the device from the thermal lesion is facilitated by leaving the bulkiest part, i.e. the expandable membrane, "in situ" thus remarkably simplifying the operation.

A further advantage of the device and the method for the TA is that they are usable with electromagnetic energy both in the radiofrequencies and the microwaves range, with little manufacturing differences which will be promptly evident to those skilled in the art.

This and other advantages of the device for TA according to the present invention will be evident to those skilled in the art from the following detailed description of some embodiments thereof with reference to the annexed drawings wherein:
Figure 1 shows a sectional detailed view of the end of the hollow element of a first embodiment of the device for the TA;
Figure 2 shows a sectional detailed view of the end of the hollow element of a second embodiment of the device for the TA;
Figure 3 shows a sectional detailed view of the end of the hollow element of a third embodiment of the device for the TA;
Figure 4 shows a sectional detailed view of the end of the hollow element of a fourth embodiment of the device for the TA;
Figure 5 shows a sectional detailed view of the end of the hollow element of a fifth embodiment of the device for the TA; and
Figure 6 shows a sectional detailed view of the end of the hollow element of a sixth embodiment of the device for the TA.

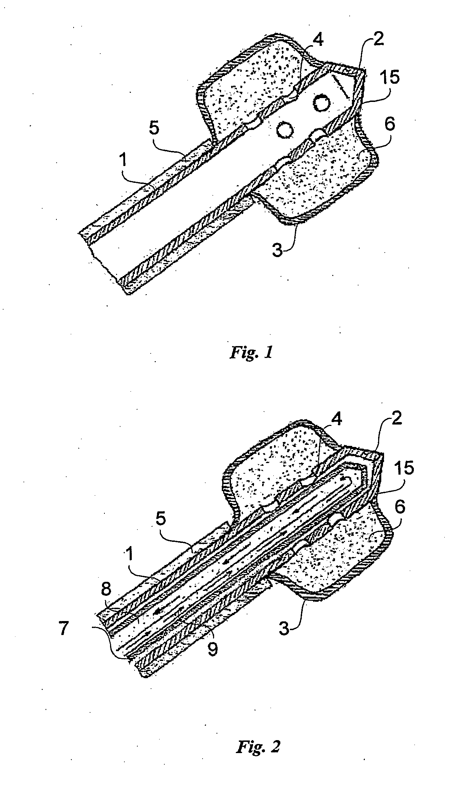

Fig. 1 shows cross section of the device for the TA according to a first embodiment of the invention. The device includes a thin hollow element 1, such for instance a needle or a catheter, with a closed tip 2, suitable for penetrating the tissues to be subject to the TA procedure. The device is provided with an expandable and semipermeable membrane 3, wherein the hollow element 1 is coaxially inserted and sealed. In this particular embodiment, the hollow element 1 is made of a conductive material and it is connected to a radiofrequency energy generator. Thus in this embodiment the hollow element 1 is the active electrode of the TA device. The hollow element 1 is provided with one or more openings 4 circumferentially arranged in proximity to its tip 2. The end of the hollow element 1 is also surrounded by the membrane 3 that is sealed thereon. The residual portion of the hollow element 1 can be insulated, for example, by means of an insulating paint or an insulating sheath 5. Once inserted the hollow element 1 in the tumoral mass, an injection system injects a substance 6 through the opening or openings 4 of the hollow element 1 into the membrane 3, the substance 6 expands the membrane 3 thus generating on the tissues a pressure being higher than the atmospheric one, and permeates there-through, thus closely contacting the surrounding tumoral tissues. Then the generator delivers electromagnetic energy, thus causing ionic turbulence in the zone surrounding the element 1 and thereby resistive heat. The transmission of the energy to the tissues is carried out due the electric conductivity properties of the substance 6, which contacts the hollow element 1. All the tissues being comprised between the electrodes and the 60°C isotherm undergo a non-reversible coagulative necrosis. Non-reversible damages are associated to temperatures comprised between 46°C and 60°C, whose entity is proportional to the time of exposure.

The substance 6 must be biocompatible and capable of maintaining a low coupling impedance between the active part of the device and the tumoral tissues even at high temperatures. In such a way a continuous energy delivery from the device to the tissues is granted. In fact, as it may be learnt from patent publication WO 2007/113866 in the name of the same applicant, the injection into the tumoral mass of an electrically conductive substance, being capable of maintaining hydrated the region surrounding the electrode even at very high temperatures and/or maintaining the impedance constantly low during the energy delivery, allows to extend such a delivery for a very long time and thereby to generate thermal lesions having a large size without reaching the dehydration and the carbonization of the same tissues. Thereby it is possible to predict the size of the thermal lesion by setting a suitable time-profile of the power delivery.

Still in the patent publication WO 2007/113866, it may be learnt that the substance 6 is biocompatible, dries or boils at temperatures being higher than the boiling temperature of the tissue liquids, has a viscosity being higher than that of the blood and has an electric conductivity comprised between one tenth and one hundred times the electric conductivity of the tissue liquids. The substance 6 may be a gel, a hydrogel, a thixotropic hydrogel, an aqueous ionic solution, a suspension having a size of the suspended particles comprised between about 1 µm and about 1000 µm, or a mixture of such substances.

One of the main characteristics of the invention is that the retaining action of the membrane 3 allows to keep the distribution of the substance 6 through the tissues totally under control, the substance permeating through the membrane 3 reaching the external surface thereof thus closely contacting the surrounding tissues. The possibility of exactly controlling the distribution of the substance 6, allows to surely predict the shape of the generated thermal lesion. The membrane 3 may have any shape, however in the preferred embodiments a cylindrical geometry is used with suitable zones connecting it to the hollow element 1.

Suitable materials for the manufacturing of the semipermeable membrane are, for example, the biological membranes, or woven or non-woven polymeric materials based on PET, PP, PA or PE.

Another characteristic of the device according to the present invention is that due to the effect of the injection of the substance 6 into the membrane 3, the local pressure on the tissues increases over the atmospheric pressure. As it may be learnt from a patent publication WO 2007/113865 in the name of the same applicant, the boiling temperature increase in the tissue liquids, being due to the pressure locally exerted by an expandable membrane, allows to deliver more energy to the tissues and thereby to generate thermal lesions having dimensions that are larger than those obtainable with known techniques.

The pressure inside the membrane 3 can be measured, for instance, by means of a pressure transducer and controlled in a close loop in order to grant the maintenance of the pre-set conditions for the whole duration of the procedure.

In Fig. 2 a second embodiment of the device for TA with RF is shown according to the present invention. The design of the device is completely analogous to that of the device shown in Fig. 1, however this embodiment provides for the use of a cooling circuit being inserted into the hollow element 1, allowing to keep under control the temperature of the hollow element 1 during the treatment. In fact, as it is known the flow of electrical current generates resistive heat and the temperature profile of the heated zone has the maximum values close to the hollow element 1. The temperature control combined with the use of the substance 6 supports the duration of the TA procedures of and further increases the possibilities of setting the time-profile of the power. In the shown embodiment, the cooling circuit is composed of a small diameter canalization 7 being coaxially inserted into the hollow element 1. A conventional pumping system circulates a cooling substance 8 in the canalization 7, absorbing heat from the end of the element 1 and releasing it by passing, for instance, through a heat exchanger and then returning towards the end of the hollow element 1. The arrows 9 indicate an hypothetical circulation direction of the cooling substance 8 inside the canalization 7.

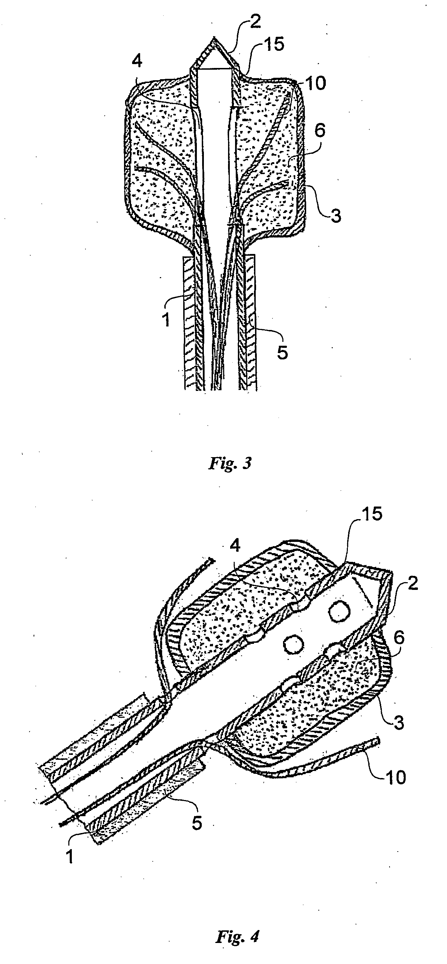

In Fig. 3 a third embodiment of the device for the TA with RF is shown according to the present invention. The design of the element 1 and of the membrane 3 is analogous to that of the previous drawings, however in this case the openings 4 provided in the proximity of the tip 2 of the hollow element 1 have a large size in order to allow the extraction of one or more filiform electrodes 10 in the space comprised between the hollow element 1 and the membrane 3. The electrodes 10 improve the energy delivery distribution as they increase the electrode surface thus allowing to further increase the efficiency of delivery of electromagnetic energy.

Fig. 4 shows a fourth embodiment of the device for the TA with RF according to the present invention, being analogous to the one shown in Fig. 3. In this case the filiform electrodes 10 are extracted from the hollow element 1 at the outside of the membrane 3 and contact the tissues. In other embodiments (not shown) it is also possible to combine filiform electrodes 10 inside and outside the membrane 3.

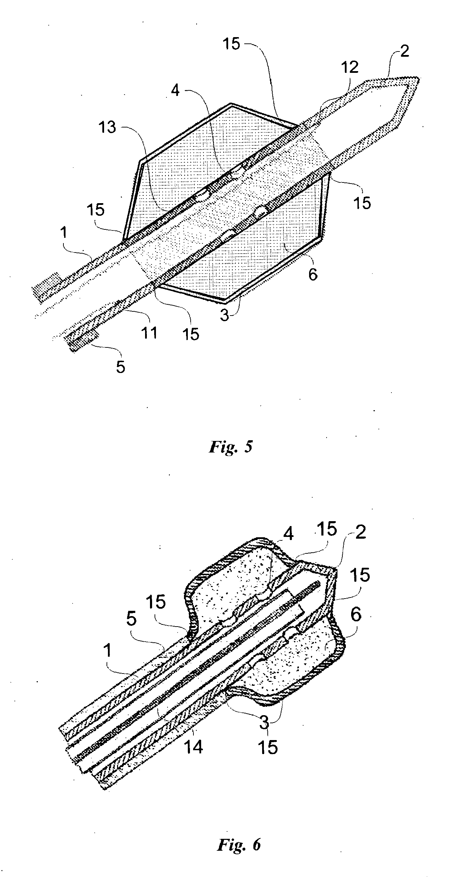

Fig. 5 shows a fifth embodiment of the device for TA with RF according to the present invention, using a bipolar technique for the delivery of electromagnetic energy. The end of the hollow element 1 enclosed in the membrane 3 is divided into an upper zone 11 and a lower zone 12 by interposing a ring 13 being made of an insulating material and having diameter and thickness equal to the hollow element 1. The two upper 11 and lower 12 zones are connected to the two poles of the circuit and form the active electrode and the counter electrode, respectively. In a TA procedure the substance 6 is injected into the membrane 3 through the openings 4 of the hollow element 1 as previously described. When switching on the generator, electromagnetic field lines are generated going from one electrode to the other one and causing, as in the previous cases, ionic turbulence and consequent resistive heat.

Fig. 6 shows a sixth embodiment of the device for the TA according to the present invention of a microwaves type, wherein, in the same way as in the previous embodiments, the hollow element 1 is provided with a membrane 3 and with one or more openings 4 circumferentially arranged in proximity of the tip 2 of the hollow element 1. In this embodiment, differently from the previous ones, inside the hollow element 1 a coaxial cable 14 is arranged, delivering electromagnetic energy in the microwaves range. In this case the hollow element 1 is formed by materials being transparent to the microwaves in order not to interfere with their propagation through the tissues.

A further characteristic of the device and the method according to the present invention is that, once completed the TA procedure, the membrane 3 can be left in situ, that is in the necrotized tissue mass. The possibility of leaving the membrane in situ leads to a remarkable simplification of the procedure, which only requires the extraction of the hollow element 1 from the patient's body once it is ended. This does not affect the patient's health, as the membrane material is absolutely biocompatible as well as the substance 6 used to expand it.

The detachment of the hollow element 1 from the membrane 3 occurs in correspondence to connection areas 15 provided on the hollow element 1 by applying a predetermined load. For instance connection and release of the membrane could be accomplished by a gluing with a pre-set releasing load, by screwing and unscrewing rotating the catheter body on threaded corresponding profiles, or by snapping.

By means of the above-described devices it is possible to perform the TA method according to the present invention, comprising the steps of:
a. inserting a device into a tumoral mass, being provided with a hollow element 1 that is tightly inserted into an expandable membrane 3;
b. pressurizing said membrane 3 by injecting a substance 6; and
c. delivering electromagnetic energy at a high frequency in the tumoral mass till the coagulative necrosis of the tissues.
The method provides for leaving the expandable membrane 3 in situ, that is inside the necrotized tissue, at the end of the TA treatment.

## Claims

1. A device for the thermal ablation comprising a thin hollow element (1) suitable for being connected to an electromagnetic energy generator at high frequency, an expandable membrane (3) made of biocompatible and semipermeable material, the membrane defining an interior space and being connected to said hollow element (1), and a substance (6) suitable for being injected into said interior space (3) through one or more openings (4) provided on the portion of the hollow element (1) being enclosed in the membrane (3), **characterized in that** said substance (6) is biocompatible, dries or boils at temperatures higher than the boiling temperature of the tissue liquids, has a viscosity higher than that of the blood and has an electric conductivity comprised between one tenth and one hundred times the electric conductivity of the tissue liquids, the membrane (3) being permeable to the substance (6).

2. A device according to claim 1, **characterized in that** the membrane (3) is made of a biological material.

3. A device according to claim 1, **characterized in that** the membrane (3) is made of a woven or non-woven fabric based on PET, PP, PA and/or PE.

4. A device according to one of claims 1 to 3, **characterized in that** the substance (6) is in the form of a gel.

5. A device according to one of claims 1 to 3, **characterized in that** the substance (6) is in the form of a hydrogel.

6. A device according to one of claims 1 to 3, **characterized in that** the substance (6) is in the form of a thixotropic hydrogel.

7. A device according to one of claims 1 to 3, **characterized in that** the substance (6) is an aqueous ionic solution.

8. A device according to one of claims 1 to 3, **characterized in that** the substance (6) is in the form of a suspension having a suspended particles size comprised between about 1 µm and about 1000 µm.

9. A device according to one of claims 1 to 3, **characterized in that** the substance (6) is a mixture of two or more of any of the substances (6) according to claims 4, 5, 6, 7 or 8.

10. A device according to one of the preceding claims, **characterized by** further comprising transducers suitable for measuring the pressure inside the membrane (3).

11. A device according to one of the preceding claims, **characterized by** further comprising a cooling circuit formed of a small diameter canalization (7) coaxially inserted into the hollow element (1) and suitable for circulating a cooling substance (8).

12. A device according to one of the preceding claims, **characterized by** further comprising one or more filiform electrodes (10) extractable from the hollow element (1) through said openings (4) provided on the portion of the hollow element (1) connected to said membrane (3).

13. A device according to one of the preceding claims, **characterized by** further comprising one or more filiform electrodes (10) extractable from the hollow element (1) at the outside of the membrane (3).

14. A device according to one of claims 1 to 11, **characterized in that** the end of the hollow element (1) is divided in an upper zone (11) and a lower zone (12) by a ring (13) made of an insulating material and having diameter and thickness equal to those of the hollow element (1), said upper (11) and lower (12) zones being respectively connected to the two poles of the circuit and said membrane (3) being coaxially assembled on the hollow element (1) and sealed on the ring (13).

15. A device according to one of the preceding claims, **characterized in that** the membrane (3) is detachable from the hollow element (1) in correspondence to connecting areas (15) provided on the hollow element (1).

16. A device according to claim 15, **characterized in that** the connecting areas (15) between the membrane (3) and the hollow element (1) are made of a gluing having a pre-set releasing load.

17. A device according to claim 15, **characterized in that** the connecting areas (15) between the membrane (3) and the hollow element (1) are made of corresponding threaded profiles.

18. A device according to claim 15, **characterized in that** the connecting areas (15) between the membrane (3) and the hollow element (1) are made of a snapping mechanism.

## Patentansprüche

1. Vorrichtung für die Wärmeablation, welche ein dünnes hohles Element (1) aufweist, welches geeignet ist, mit einem elektromagnetischen Hochfrequenz-Energiegenerator verbunden zu werden, eine dehnbare Membran (3) aus biokompatiblem und semipermeablem Material, wobei die Membran einen inneren Raum definiert und mit dem hohlen Element (1) verbunden ist, sowie eine Substanz (6), die geeignet ist, in den inneren Raum (3) durch eine oder mehrere Öffnungen (4) injiziert zu werden, wobei die Öffnungen an dem Teil des hohlen Elements (1) vorgesehen sind, welcher in der Membran (3) eingeschlossen ist,
**dadurch gekennzeichnet,**
**dass** die Substanz (6) biokompatibel ist, bei Temperaturen höher als die Siedetemperatur der Gewebeflüssigkeiten trocknet oder siedet, und eine Viskosität aufweist, die höher ist als die von Blut sowie eine elektrische Leitfähigkeit, die zwischen einem Zehntel und dem Hundertfachen der elektrischen Leitfähigkeit der Gewebeflüssigkeiten liegt, wobei die Membran (3) für die Substanz (6) durchlässig ist.

2. Vorrichtung nach Anspruch 1,
**dadurch gekennzeichnet,**
**dass** die Membran (3) aus einem biologischen Material gefertigt ist.

3. Vorrichtung nach Anspruch 1,
**dadurch gekennzeichnet,**
**dass** die Membran (3) aus einem gewebten oder nicht gewebten Stoff auf der Basis von PET, PP, PA und/oder PE gefertigt ist.

4. Vorrichtung nach einem der Ansprüche 1 bis 3,
**dadurch gekennzeichnet,**
**dass** die Substanz (6) in Form eines Gels vorliegt.

5. Vorrichtung nach einem der Ansprüche 1 bis 3,
**dadurch gekennzeichnet,**
**dass** die Substanz (6) in Form eines Hydrogels vorliegt.

6. Vorrichtung nach einem der Ansprüche 1 bis 3,
**dadurch gekennzeichnet,**
**dass** die Substanz (6) in Form eines tixotropischen Hydrogels vorliegt.

7. Vorrichtung nach einem der Ansprüche 1 bis 3,
**dadurch gekennzeichnet,**
**dass** die Substanz (6) eine wässrige ionische Lösung ist.

8. Vorrichtung nach einem der Ansprüche 1 bis 3,
**dadurch gekennzeichnet,**
**dass** die Substanz (6) in Form einer Suspension vorliegt, wobei die suspendierten Partikel eine Größe aufweisen, die zwischen ungefähr 1 µm und ungefähr 1000µm liegt.

9. Vorrichtung nach einem der Ansprüche 1 bis 3,
**dadurch gekennzeichnet,**
**dass** die Substanz (6) eine Mischung von 2 oder mehr der Substanzen (6) gemäß den Ansprüchen 4, 5, 6, 7 oder 8 ist.

10. Vorrichtung nach einem der vorhergehenden Ansprüche, **gekennzeichnet durch** Wandler, die zur Messung des Druckes innerhalb der Membran (3) geeignet sind.

11. Vorrichtung nach einem der vorhergehenden Ansprüche, **gekennzeichnet durch** einen Kühlkreislauf, welcher aus einer Kanalisation (7) mit kleinem Durchmesser gebildet ist, der koaxial in das hohle Element (1) eingesetzt ist und der geeignet ist, die Kühlsubstanz (8) zu zirkulieren.

12. Vorrichtung nach einem der vorhergehenden Ansprüche, **gekennzeichnet durch** eine oder mehrere fadenförmige Elektroden (10), welche aus dem hohlen Element (1) **durch** die Öffnungen (4) herausziehbar sind, welche auf dem Teil des hohlen Elements (1) vorgesehen sind, der mit der Membrane (3) verbunden ist.

13. Vorrichtung nach einem der vorhergehenden Ansprüche, **gekennzeichnet durch** eine oder mehrere fadenförmige Elektroden (10), welche aus dem hohlen Element (1) an der Außenseite der Membrane (3) herausziehbar sind.

14. Vorrichtung nach einem der Ansprüche 1 bis 11,
**dadurch gekennzeichnet,**
**dass** das Ende des hohlen Elements (1) in eine obere Zone (11) und eine untere Zone (12) durch einen Ring (13) unterteilt ist, welcher aus einem isolierenden Material gefertigt ist und einen Durchmesser und eine Dicke aufweist, die gleich ist der des hohlen Elements (1), wobei die obere (11) und die untere (12) Zone jeweils mit zwei Polen des Kreises verbunden sind und die Membran (3) koaxial am hohlen Element (1) montiert und auf dem Ring (3) abgedichtet ist.

15. Vorrichtung nach einem der vorhergehenden Ansprüche,
**dadurch gekennzeichnet,**
**dass** die Membran (3) von dem hohlen Element (1) abtrennbar ist mit entsprechenden Verbindungsflächen 15, die auf dem hohlen Element (1) vorgesehen sind.

16. Vorrichtung nach Anspruch 15,
**dadurch gekennzeichnet,**
**dass** die Verbindungsflächen (15) zwischen der Membran (3) und dem hohlen Element (1) aus einem Klebstoff gemacht sind, der eine voreinstellbare Ablösekraft aufweist.

17. Vorrichtung nach Anspruch 15,
**dadurch gekennzeichnet,**
**dass** die Verbindungsflächen (15) zwischen der Membran (3) und dem hohlen Element (1) aus miteinander korrespondierenden Gewindeprofilen gemacht sind.

18. Vorrichtung nach Anspruch 15,
**dadurch gekennzeichnet,**
**dass** die Verbindungsflächen (15) zwischen der Membran (3) und dem hohlen Element (1) aus einem Schnappmechanismus gemacht sind.

## Revendications

1. Dispositif d'ablation thermique comprenant un élément creux fin (1) adapté pour être connecté à un générateur d'énergie électromagnétique haute fréquence, une membrane extensible (3) faite en un matériau biocompatible et semi-perméable, la membrane définissant un espace intérieur, et étant connectée audit élément creux (1), et une substance (6) adaptée pour être injectée dans ledit espace intérieur (3) par l'intermédiaire d'une ou plusieurs ouvertures (4) disposées sur la partie de l'élément creux (1) enfermée dans la membrane (3), **caractérisé en ce que** ladite substance (6) est biocompatible, sèche ou bout à des températures supérieures au point d'ébullition des liquides tissulaires, a une viscosité supérieure à celle du sang et a une conductivité électrique comprise entre un dixième et cent fois la conductivité électrique des liquides tissulaires, la membrane (3) étant perméable à la substance (6).

2. Dispositif selon la revendication 1, **caractérisé en ce que** la membrane (3) est faite en un matériau biologique.

3. Dispositif selon la revendication 1, **caractérisé en ce que** la membrane (3) est faite en une étoffe tissée ou non-tissée à base de PET, PP, PA et / ou PE.

4. Dispositif selon l'une des revendications 1 à 3, **caractérisé en ce que** la substance (6) est sous la forme d'un gel.

5. Dispositif selon l'une des revendications 1 à 3, **caractérisé en ce que** la substance (6) est sous la forme d'un hydrogel.

6. Dispositif selon l'une des revendications 1 à 3, **caractérisé en ce que** la substance (6) est sous la forme d'un hydrogel thixotrope.

7. Dispositif selon l'une des revendications 1 à 3, **caractérisé en ce que** la substance (6) est une solution aqueuse ionique.

8. Dispositif selon l'une des revendications 1 à 3, **caractérisé en ce que** la substance (6) est sous la forme d'une suspension dont la taille des particules en suspension est comprise entre environ 1 µm et environ 1000 µm.

9. Dispositif selon l'une des revendications 1 à 3, **caractérisé en ce que** la substance (6) est un mélange de deux ou plus de deux substances (6) quelconques selon la revendication 4, 5, 6, 7 ou 8.

10. Dispositif selon l'une des revendications précédentes, **caractérisé en ce qu'**il comprend en outre des transducteurs adaptés pour mesurer la pression à l'intérieur de la membrane (3).

11. Dispositif selon l'une des revendications précédentes, **caractérisé en ce qu'**il comprend en outre un circuit de refroidissement formé d'une canalisation de petit diamètre (7) insérée coaxialement dans l'élément creux (1) et adapté pour faire circuler une substance de refroidissement (8).

12. Dispositif selon l'une des revendications précédentes, **caractérisé en ce qu'**il comprend en outre une ou plusieurs électrodes filiformes (10) extractibles hors de l'élément creux (1) par l'intermédiaire desdites ouvertures (4) disposées sur la partie de l'élément creux (1) connectée à ladite membrane (3).

13. Dispositif selon l'une des revendications précédentes, **caractérisé en ce qu'**il comprend en outre une ou plusieurs électrodes filiformes (10) extractibles hors de l'élément creux (1) à l'extérieur de la membrane (3).

14. Dispositif selon l'une des revendications 1 à 11, **caractérisé en ce que** l'extrémité de l'élément creux (1) est divisée en une zone supérieure (11) et une zone inférieure (12) par un anneau (13) fait en un matériau isolant et ayant un diamètre et une épaisseur identiques à ceux de l'élément creux (1), lesdites zones supérieure (11) et inférieure (12) étant respectivement connectées aux deux pôles du circuit et ladite membrane (3) étant coaxialement assemblée sur l'élément creux (1) et scellée sur l'anneau (13).

15. Dispositif selon l'une des revendications précédentes, **caractérisé en ce que** la membrane (3) est détachable de l'élément creux (1) en correspondance avec des zones de connexion (15) disposées sur l'élément creux (1).

16. Dispositif selon la revendication 15, **caractérisé en ce que** les zones de connexion (15) entre la membrane (3) et l'élément creux (1) sont réalisées par un collage ayant une force de décollage présélectionnée.

17. Dispositif selon la revendication 15, **caractérisé en ce que** les zones de connexion (15) entre la membrane (3) et l'élément creux (1) sont faites de profils filetés correspondants.

18. Dispositif selon la revendication 15, **caractérisé en ce que** les zones de connexion (15) entre la membrane (3) et l'élément creux (1) sont réalisées par un mécanisme à fermoir.
